# FASCICULE DE BREVET EUROPEEN

(11) **EP 0 737 248 B1**
(45) Date de publication et mention de la délivrance du brevet: **14.11.2007**
(21) Numéro de dépôt: 95905172.3
(22) Date de dépôt: 27.12.1994
(51) Int. Cl.: C12N 15/11, C12N 15/86, C07H 21/02, A61K 31/70

(54) **CONTROLE DE L'EXPRESSION DU GENE DE L'IGF-I**
KONTROLLE DER EXPRIMIERUNG DES IGF-I GENS
CONTROL OF THE EXPRESSION OF THE IGF-I GENE

(30) Priorité: 29.12.1993 FR 9315798
(43) Date de publication de la demande: 16.10.1996
(60) Demande divisionnaire: 07007057.8
(73) Titulaire: CENTRE NATIONAL DE LA RECHERCHE SCIENTIFIQUE (CNRS), 75016 Paris (FR); INSTITUT NATIONAL DE LA SANTE ET DE LA RECHERCHE MEDICALE (INSERM), 75654 Paris Cédex 13 (FR); MUSEUM NATIONAL D'HISTOIRE NATURELLE, 75005 Paris (FR)
(72) Inventeur: GIOVANNANGELI, Carine, F-75005 Paris (FR); HELENE, Claude, F-75004 Paris (FR)
(74) Mandataire: Savina, Jacques
(86) Numéro de dépôt international: PCT/FR1994/001536
(87) Numéro de publication internationale: WO 1995/018223

(56) Documents cités:
- WO-A-91/06626
- WO-A-92/19732
- WO-A-93/12230
- FR-A- 2 687 411
- PROCEEDINGS OF THE NATIONAL ACADEMY OF SCIENCES OF THE UNITED STATES OF AMERICA 90 (21). 1993. 10013-10017, GIOVANNANGELI, C. ET AL. 'Oligonucleotide clamps arrest DNA synthesis on a single - stranded DNA target.'
- J AM CHEM SOC 113 (20). 1991. 7775-7777, GIOVANNANGELI, C. ET AL. 'SINGLE - STRANDED DNA AS A TARGET FOR TRIPLE - HELIX FORMATION.' cité dans la demande
- PURE & APPLIED CHEMISTRY, vol. 66,no. 4, 1994 pages 663-669, HELENE, C. 'Rational design of sequence-specific DNA ligands for artificial control of gene expression'
- GENE, vol. 149, 4 Novembre 1994 AMSTERDAM NL, pages 115-121, KANDIMALLA, E. & AGRAWAL, S. 'Single-stranded-targeted triplex formation: stability, specificity and RNase H activation properties '
- ROBERTS R.; CROTHERS D.: 'Stability and properties of double and triple helices: dramatic effects of RNA or DNA backbone composition' SCIENCE vol. 258, 27 Novembre 1992, pages 1463 - 1466, XP002026847
- MILLIGAN J. ET AL: 'Oligoribonucleotide synthesis using T7 RNA polymerase and synthetic DNA duplexes' NUCLEIC ACIDS RESEARCH vol. 15, 1987, pages 8783 - 8798, XP002360370
- LANDRETH K. ET AL: 'Insulin-like growth factor-I regulates pro-B cell differentiation' BLOOD vol. 80, 01 Septembre 1992, pages 1207 - 1212
- MOATS-STAATS B. ET AL: 'Insulin-like growth factor-I (IGF-I) antisense oligodeoxynucleotide mediated inhibition of DNA synthesis by WI-38 cells: evidence for autocrine actions of IGF-I' MOLECULAR ENDOCRINOLOGY vol. 7, 1993, pages 171 - 180

## Description

La présente invention concerne de nouveaux vecteurs, les compositions pharmaceutiques les contenant, et leurs utilisations thérapeutiques. Plus particulièrement, elle concerne de nouveaux vecteurs permettant d'agir, de manière très sélective et très efficace, sur l'expression de gènes.

La régulation de l'expression de gènes-cible au moyen d'oligonucléotides constitue une approche thérapeutique en développement croissant. Cette approche repose sur la capacité des oligonucléotides à s'hybrider spécifiquement à des régions complémentaires d'un acide nucléique, et à inhiber ainsi spécifiquement l'expression de gènes déterminés. Cette inhibition peut intervenir soit au niveau traductionnel (oligonucléotide anti-sens) soit au niveau transcriptionnel (oligonucléotide anti-gène).

Les oligonucléotides anti-sens sont des séquences nucléiques capables de s'hybrider sélectivement avec des ARNs messager cellulaires-cible, pour inhiber leur traduction en protéine. Ces oligonucléotides forment avec l'ARNm-cible, de manière locale, des régions double-brin, de type ARN/ARNm ou même ADN/ARNm, par interaction de type Watson-Crick classique. Il peut s'agir par exemple d'oligonucléotides synthétiques, de petite taille, complémentaires d'ARNm cellulaires, et qui sont introduits dans les cellules-cible. De tels oligonucléotides ont par exemple été décrits dans le brevet n° EP 92 574. Il peut également s'agir de gènes anti-sens dont l'expression dans la cellule-cible génère des ARN complémentaires d'ARNm cellulaires. De tels gènes ont par exemple été décrits dans le brevet n° EP 140 308. Molecular Endocrinology, p 172 vol 7 (2) 1993., Blood vol 80 (5) 1992, pp 1207-1212.

Plus récemment, un nouveau type d'oligonucléotides capables de réguler l'expression de gènes-cible a été mis en évidence. Ces oligonucléotides ne s'hybrident pas avec les ARNm cellulaires, mais directement avec l'ADN génomique en double-brin. Cette nouvelle approche repose sur la mise en évidence que certains oligonucléotides sont capables d'interagir spécifiquement dans le grand sillon de la double-hélice d'ADN pour former localement des triple-hélices, conduisant à une inhibition de la transcription de gènes-cible. Ces oligonucléotides reconnaissent sélectivement la double-hélice d'ADN au niveau de séquences oligopurine.oligopyrimidine, c'est-à-dire au niveau de régions possédant une séquence oligopurique sur un brin et une séquence oligopyrimidique sur le brin complémentaire, et y forment localement une triple-hélice. Les bases du troisième brin (l'oligonucléotide) forment des liaisons hydrogène (liaisons Hoogsteen ou Hoogsteen inverse) avec les purines des paires de bases Watson-Crick. Les oligonucléotides anti-gène peuvent contenir les bases suivantes :
- thymidine (T), qui est capable de former des triplets avec les doublets AT de l'ADN double-brin (Rajagopal et al, Biochem 28 (1989) 7859);
- adénine (A), qui est capable de former des triplets avec les doublets AT de l'ADN double-brin;
- guanine (G), qui est capable de former des triplets avec les doublets G.C de l'ADN double-brin;
- cytosine protonée (C+), qui est capable de former des triplets avec les doublets G.C de l'ADN double-brin (Rajagopal et al précitée);

Des oligonucléotides anti-gène ont notamment été décrits par Hélène dans Anti-Cancer drug design 6 (1991) 569. PNAS 90 pp 10013-10017, (1993).

La présente invention décrit une nouvelle approche pour le contrôle de l'expression de gènes-cible. Elle réside plus particulièrement dans la mise en évidence qu'il est possible de produire génétiquement *in vivo* des ARN thérapeutiques capables de former des triple-hélices avec des acides nucléiques-cible simple-brin. Préférentiellement, elle réside dans la production génétique *in vivo* d'ARN thérapeutiques capables de former des triple-hélices avec des cibles ribonucléiques.

L'invention concerne notamment les séquences d'ADN double-brin codant pour de tels ARN thérapeutiques. Elle concerne également les vecteurs utilisables pour la production *in vivo* de ces ARN thérapeutiques, notamment dans le cadre d'une thérapie génique. Elle concerne aussi les compositions pharmaceutiques comprenant ces ADN double-brin ou ces vecteurs.

Un autre aspect de l'invention réside dans un procédé de régulation de l'expression de gènes cellulaires par transformation de cellules-cible au moyen d'un vecteur tel que mentionné plus haut

Plus particulièrement, la présente invention concerne donc un ADN double-brin codant pour un ARN capable de former une triple-hélice avec un acide nucléique cible simple-brin.

Les ADN double-brin de l'invention codent plus particulièrement pour des ARN composites comprennant au moins :
- une première région capable de former une double-hélice avec l'acide nucléique simple-brin ciblé ou avec une partie de celui-ci,
- une deuxième région capable de former une triple-hélice avec la double-hélice ainsi formée ou avec une partie de celle-ci, et,
- un ou deux bras reliant les deux régions, chacune des régions pouvant être continue ou interrompue.

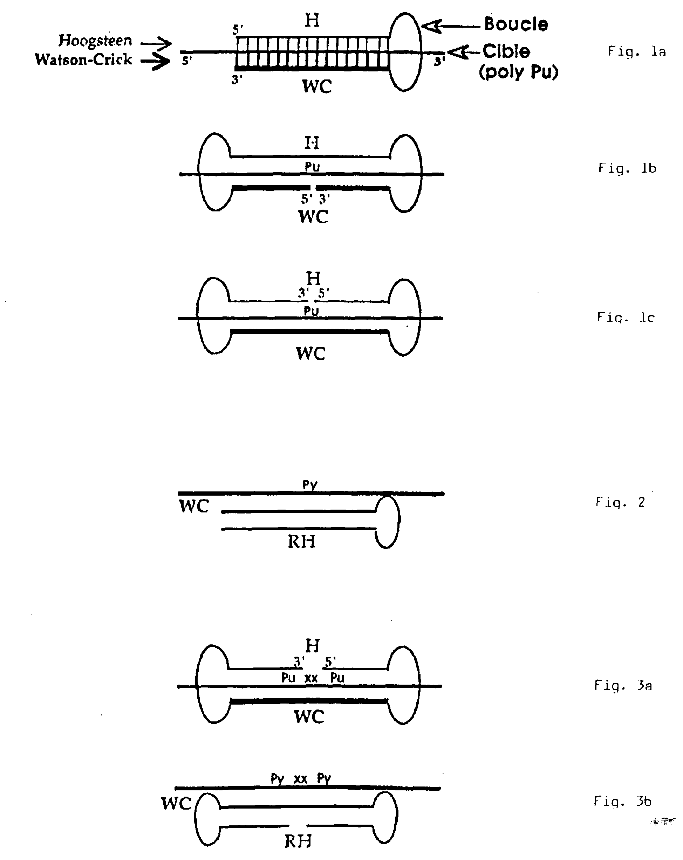

Dans un premier mode particulier de mise en oeuvre de l'invention, la première région de l'ARN composite forme avec l'acide nucléique simple-brin ciblé une double-hélice par interaction de type Watson-Crick classique, puis la deuxième région forme avec cette double-hélice ou avec une partie de celle-ci une triple-hélice par liaisons hydrogène (de type Hoogsteen ou Hoogsteen inverse) (voir figures 1-4).

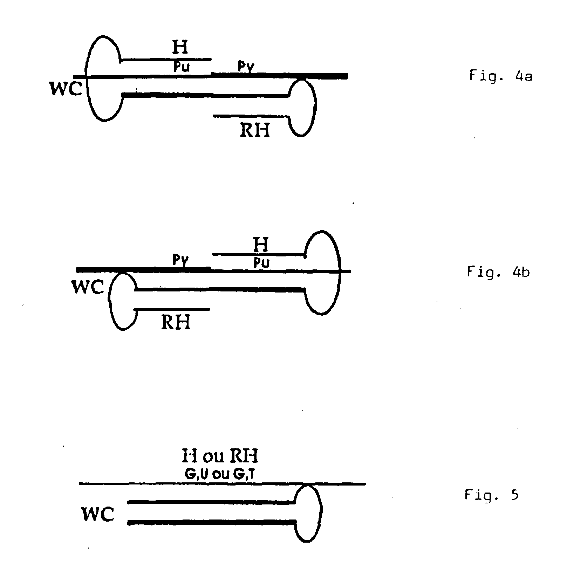

Dans un autre mode particulier de mise en oeuvre de l'invention, les deux régions de l'ARN composite de l'invention forment entre elles une double-hélice par appariement de type Watson-Crick, laquelle double-hélice interagit avec l'acide nucléique simple-brin ciblé par liaisons hydrogène (de type Hoogsteen ou Hoogsteen inverse), formant ainsi une triple-hélice (voir figure 5).

L'acide nucléique ciblé simple-brin peut être un ARN, tel que par exemple un ARN messager (ARNm), un ARN de transfert (ARNt), un ARN ribosomique (ARNr) ou un ARN viral. Il peut également s'agir d'un ADN simple-brin, apparaissant par exemple lors de la réplication de l'ADN double-brin ou d'une région de l'ADN localement ouverte par nature.

Plus particulièrement, les ARN de l'invention sont capables de former des triple-hélices avec les acides nucléiques simple-brin ciblés, au niveau de régions constituées préférentiellement de bases puriques (A et G : région polyPu), ou de bases pyrimidiques (T/U et C : région polyPy), ou de bases T/U et G (région polyT/U, G).

Ainsi, lorsque l'acide nucléique ciblé (ou une région déterminée de celui-ci) est composé essentiellement de bases puriques (polyPu), l'ARN thérapeutique de l'invention peut comprendre une première région composée des bases pyrimidiques complémentaires (région Watson-Crick), un ou deux bras oligonucléotidiques, et une deuxième région (région Hoogsteen) composée des bases U, C et/ou G, qui interagissent avec les bases puriques de la double-hélice formée selon l'appariement mentionné ci-après : les bases C et G sont capables de former des liaisons hydrogène avec la guanine des doublets G.C; la base U est capable de former des liaisons hydrogène avec l'adénine des doublets A.T ou A.U.

Dans ce mode de réalisation, l'ARN thérapeutique de l'invention forme une boucle autour de l'acide nucléique-cible ou une partie de celui-ci, selon la configuration représentée sur la figure 1a.

Lorsque l'acide nucléique-cible (ou une région déterminée de celui-ci) est composé essentiellement de bases pyrimidiques (polyPy), l'ARN thérapeutique de l'invention peut comprendre une première région composée des bases puriques complémentaires (région Watson-Crick), un ou deux bras oligonucléotidiques, et une deuxième région (région Hoogsteen) composée des bases U et G ou A et G, qui interagissent avec les bases puriques de la double-hélice formée selon l'appariement mentionné ci-après : la base G est capable de former des liaisons hydrogène (Hoogsteen inverse) avec la guanine des doublets G.C; les bases U et A sont capables de former des liaisons hydrogène avec l'adénine des doublets A.T ou A.U.

Dans ce mode de réalisation, l'ARN thérapeutique de l'invention forme une boucle parallèle à l'acide nucléique-cible, selon la configuration représentée sur la figure 2.

Lorsque l'acide nucléique-cible (ou une région déterminée de celui-ci) est composé essentiellement de bases T/U,C ou T/U,G, l'ARN thérapeutique de l'invention peut comprendre une première région composée des bases puriques complémentaires (région Watson-Crick), un ou deux bras oligonucléotidiques, et une deuxième région composée des bases pyrimidiques complémentaires des bases puriques (région également Watson-Crick), les 2 régions de l'ARN thétapeutique formant une double-hélice Watson-Crick dont le brin purique interagit avec l'acide nucléique-cible, par formation de liaisons Hoogsteen ou Hoogsteen inverse.

Dans ce mode de réalisation, l'ARN thérapeutique de l'invention forme une boucle parallèle à l'acide nucléique-cible, selon la configuration représentée sur la figure 5.

Il est entendu que, selon la composition de l'acide nucléique ciblé simple-brin choisie, l'homme du métier peut définir d'autres séquences d'ADN double-brin de l'invention, basées sur l'appariement des doublets Watson-Crick et Hoogsteen ou Hoogsteen inverse.

Le ou les bras reliant les deux régions des ARN thérapeutiques de l'invention sont des séquences oligonucléotidiques qui peuvent comporter toute base composant les ARN (A, U, G ou C). La séquence du bras ne doit pas, de préférence, être susceptible de s'apparier elle-même avec l'acide nucléique ciblé, pour ne pas perturber la formation de la triple-hélice locale. La longueur de ce bras peut être adaptée par l'homme du métier en fonction de l'acide nucléique ciblé. Généralement, elle est comprise entre 3 et 6 bases, de préférence entre 3 et 5.

Par ailleurs, dans un mode particulier de réalisation, les ADN double-brin de l'invention peuvent coder pour des ARN composites comprenant 2 bras oligonucléotidiques permettant la formation de deux boucles autour de, et/ou, parallèles à, l'acide nucléique ciblé simple-brin. De tels ARN composites présentent des propriétés particulièrement avantageuses :
- Ils permettent tout d'abord d'augmenter encore la stabilité de la triple-hélice formée, ainsi que son efficacité thérapeutique, en raison d'un encombrement stérique encore plus important (Cf figures 1b, 1c, 3 et 4).
- Ils permettent de cibler des acides nucléiques simple-brin au niveau de régions oligopuriques ou oligopyrimidiques interrompues respectivement par des bases pyrimidiques ou puriques, en interrompant la région Hoogsteen ou Hoogsteen inverse (Cf figures 3a et 3b). Ceci permet donc d'élargir le champ d'application de ces ARN thérapeutiques à tout type de région d'un acide nucléique simple-brin.
- Ils permettent également de cibler des acides nucléiques simple-brin composés d'un enchaînement d'une région oligopurique et d'une région oligopyrimidique, et inversement (figure 4). Ceci élargit donc encore le champ d'application des ARN thérapeutiques de l'invention.

Dans un mode de réalisation particulier de l'invention, l'ADN double-brin code donc pour un ARN composite comprenant :
- une première région capable de former une double-hélice avec l'acide nucléique simple-brin ciblé ou avec une partie de celui-ci,
- une deuxième région capable de former une triple-hélice avec la double-hélice ainsi formée ou avec une partie de celle-ci, et,
- 2 bras reliant chacune des deux régions à leurs extrémités (figures 1b, 1c, 3 et 4).

Dans ce mode de réalisation, l'une des 2 régions de l'ARN composite (Watson-crick (figure 1b), Hoogsteen (figure 1c et 3a) ou Hoogsteen inverse (figure 3b)) est interrompue, car elle contient le site d'initiation de la transcription.

Dans un autre mode de réalisation particulier de l'invention, l'ADN double-brin code pour un ARN composite comprenant :
- une première région capable de former une double-hélice avec une région oligopurique de l'acide nucléique simple-brin ciblé,
- un bras,
- une deuxième région capable de former une triple-hélice avec la double-hélice ainsi formée ou avec une partie de celle-ci,
- une troisième région, liée à la première, capable de former une double-hélice avec une région oligopyrimidique de l'acide nucléique simple-brin ciblé,
- un deuxième bras, et,
- une quatrième région, liée à la troisième, capable de former une triple-hélice avec la double-hélice ainsi formée ou avec une partie de celle-ci (figures 4).

De préférence, les ADN double-brin de l'invention (et les ARN composites codés possèdent une longueur supérieure à 10 bases, et, plus préférentiellement, supérieure à 15 bases. Cette longueur est adaptée par l'homme du métier en fonction de la longueur de l'acide nucléique ciblé en simple-brin, de manière à assurer la stabilité, la spécificité et la sélectivité de l'ARN thérapeutique. Par ailleurs, pour améliorer la stabilité de la triple-hélice, il peut être intéressant d'allonger l'une des régions de l'ARN composite, de préférence la région formant une double-hélice.

L'ADN double-brin peut être un ADN synthétique ou semi-synthétique. Il peut être obtenu par toute technique connue de l'homme du métier, et notamment au moyen de synthétiseurs d'acides nucléiques. La séquence de cet ADN est déterminée en fonction de la cible cellulaire choisie, comme indiqué ci-avant.

Avantageusement, l'ADN double-brin de l'invention comporte également des signaux permettant la transcription (la production) des ARN thérapeutiques de l'invention dans les cellules-cible. Ces signaux comprennent des séquences permettant l'initiation de la transcription (promoteurs) et, éventuellement, des signaux permettant l'arrêt de la transcription (terminateurs) ainsi que des signaux permettant la stabilisation des ARN (par exemple une queue polyA). Ces différents signaux peuvent être constitutifs ou régulés. Notamment, il peut s'agir de séquences promotrices de gènes eucaryotes, viraux, ou de promoteurs synthétiques. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs ElA, MLP, CMV, RSV, HIV, etc. En outre, ces séquences d'expression peuvent , être modifiées par addition de séquences d'activation, de régulation, etc. Par ailleurs, lorsque l'ADN double-brin ne comporte pas de séquences d'expression, il peut être inséré dans un vecteur, en aval d'une telle séquence. Un système d'expression particulièrement intéressant consiste dans l'utilisation de promoteurs transcriptionnels contrôlés spécifiquement par l'ARN polymérase III. L'ARN polymérase III est en effet responsable de la synthèse de petits ARN cytoplasmiques ou nucléaires présentant une stabilité élevée et qui ne sont pas traduits en protéine. Ces petits ARN sont notamment les ARNt, les ARNr, ou certains ARN viraux, tels que notamment les ARN VA de l'adénovirus. Selon un mode de réalisation avantageux de l'invention, l'ADN double-brin comprend un promoteur transcrit spécifiquement par l'ARN polymérase III. Plus particulièrement, l'ADN double-brin peut être inséré dans un gène transcrit spécifiquement par l'ARN polymérase III (FR 2,687,411) ou fusionné en aval d'un tel gène (EP 387 775).

La présente invention offre de nombreux avantages par rapport aux techniques antérieures de contrôle de l'expression de gènes. En particulier, la production *in vivo* d'ARN selon l'invention capables de former des triple-hélices avec des acides nucléiques cellulaires simple-brin permet d'augmenter la sélectivité des molécules thérapeutiques vis-à-vis de leurs cibles cellulaires. L'utilisation d'oligonucléotides comme agents thérapeutiques impose en effet une reconnaissance spécifique et sélective de la cible cellulaire. Ainsi, dans le cas de ras par exemple, l'ARNm de l'oncogène diffère de celui du protooncogène seulement par une mutation ponctuelle. Dans ce cas, il est important que l'oligonucléotide inhibe le plus efficacement possible l'expression de l'oncogène sans toutefois affecter celle du protooncogène. Il est donc capital de disposer d'agents thérapeutiques fortement discriminants. Dans le cas des antisens classiques, chaque base de la séquence-cible est reconnue par une seule base de la séquence de l'oligonucléotide, et un seul mésappariement peut n'avoir qu'une faible influence sur la formation et la stabilité du double-brin. Mais dans le cas des molécules thérapeutiques de l'invention, une double reconnaissance est nécessaire (chaque base de la séquence-cible est reconnue par 2 bases de l'ARN thérapeutique), ce qui augmente fortement le pouvoir discriminant.

Les ARN thérapeutiques produits dans le cadre de l'invention possèdent également une meilleure efficacité thérapeutique que les anti-sens classiques. En effet, l'encombrement physique généré par les molécules thérapeutiques de l'invention est plus important que dans le cas des anti-sens classiques, puisque celles-ci forment une boucle autour de la cible, capable de provoquer le décrochage d'une transcriptase inverse ou d'une ADN polymérase, et ainsi, susceptibles d'interagir au niveau de la traduction de l'ARNm en protéine, avec le ribosome en élongation, les enzymes et/ou autres facteurs impliqués.

Par ailleurs, en raison de la formation d'une triple-hélice (liaisons Watson-Crick et Hoogsteen ou Hoogsteen inverse), les molécules de l'invention forment avec les acides nucléiques ciblés des complexes plus stables que les anti-sens classiques. Cette propriété renforce encore les avantages des molécules de l'invention qui constituent donc des agents thérapeutiques particulièrement puissants.

Enfin, la possibilité de générer *in vivo* les molécules thérapeutiques selon l'invention permet .de produire ces molécules thérapeutiques à des niveaux élevés à partir d'un seul ADN double-brin (ou vecteur le contenant) introduit dans la cellule cible. De plus, elle permet de générer ces molécules directement dans les compartiments cellulaires désirés de la cellule cible.

L'ADN double-brin selon l'invention peut être utilisé tel quel, par exemple après injection à l'homme ou l'animal, pour réguler l'expression d'un gène donné. En particulier, il peut être injecté sous forme d'ADN nu selon la technique décrite dans la demande WO 90/11092. Il peut également être administré sous forme complexée, par exemple avec du DEAE-dextran (Pagano et al., J.Virol. 1 (1967) 891), avec des protéines nucléaires (Kaneda et al., Science 243 (1989) 375) ou avec des lipides (Felgner et al., PNAS 84 (1987) 7413). Il peut également être incorporé dans un vecteur tel qu'un liposome (Fraley et al., J.Biol.Chem. 255 (1980) 10431) ou une nanoparticule. Les liposomes sont des vésicules phospholipidiques comportant une phase aqueuse interne dans laquelle les acides nucléiques peuvent être encapsulés. La synthèse de liposomes et leur utilisation pour le transfert d'acides nucléiques est connue dans l'art antérieur (WO91/06309, WO92/19752, WO92/19730). Les nanoparticules sont des particules de petite dimension, généralement inférieure à 500 nm, capables de transporter ou de vectoriser un principe actif (tel qu'un acide nucléique) dans les cellules ou dans la circulation sanguine. Les nanoparticules peuvent être constituées par des polymères comportant une majorité de motifs dégradables tels que l'acide polylactique, éventuellement copolymérisé à du polyéthylène glycol. D'autres polymères utilisables dans la réalisation de nanoparticules ont été décrits dans l'art antérieur (voir par exemple EP 275 796; EP 520 889).

Préférentiellement, l'ADN double-brin de l'invention fait partie d'un vecteur. L'emploi d'un tel vecteur permet en effet d'améliorer l'efficacité de transfert de l'ADN double-brin dans les cellules-cible, et également d'augmenter sa stabilité dans lesdites cellules, ce qui permet d'obtenir un effet thérapeutique durable. Par ailleurs, l'emploi de vecteurs permet également de cibler certaines populations de cellules dans lesquelles les molécules thérapeutiques doivent être produites. De plus, il est possible d'introduire plusieurs ADN double-brin dans un même vecteur, ce qui augmente également l'efficacité du traitement.

Un autre objet de l'invention concerne donc un vecteur comprenant un ADN double-brin codant pour un ARN capable de former une triple-hélice avec un acide nucléique ciblé simple-brin.

Le vecteur utilisé peut être d'origines diverses, dès lors qu'il est capable de transformer les cellules animales, de préférence les cellules humaines. Il peut s'agir aussi bien d'un vecteur plasmidique ou viral. Dans un mode préféré de mise en oeuvre de l'invention, on utilise un vecteur viral, qui peut être choisi parmi les adénovirus, les rétrovirus, les virus adéno-associés (AAV), le virus de l'herpès, le cytomégalovirus, le virus de la vaccine, etc.

A cet égard, la présente invention a également pour objet tout virus recombinant comprenant, inséré dans son génome, un ADN double-brin codant pour un ARN capable de former une triple-hélice avec un acide nucléique cible simple-brin.

Des vecteurs dérivés des adénovirus, des rétrovirus, ou des AAV incorporant des séquences d'acides nucléiques hétérologues ont été décrits dans la littérature [Akli et al., Nature Genetics 3 (1993) 224; Stratford-Perricaudet et al., Human Gene Therapy 1 (1990) 241; EP 185 573, Levrero et al., Gene 101 (1991) 195; Le Gal la Salle et al., Science 259 (1993) 988; Roemer et Friedmann, Eur. J. Biochem. 208 (1992) 211; Dobson et al., Neuron 5 (1990) 353; Chiocca et al., New Biol. 2 (1990) 739; Miyanohara et al., New Biol. 4 (1992) 238; WO91/18088].

Avantageusement, le virus recombinant selon l'invention est un virus défectif. Le terme "virus défectif" désigne un virus incapable de se répliquer dans la cellule-cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence de l'acide nucléique double-brin de l'invention. Préférentiellement, le virus défectif conserve néanmoins les séquences de son génome qui sont nécessaires à l'encapsidation des particules virales.

Il est particulièrement avantageux d'utiliser les séquences nucléiques de l'invention sous forme incorporée à un adénovirus recombinant défectif.

Il existe en effet différents sérotypes d'adénovirus, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B. Comme indiqué ci-avant, il est tout particulièrement avantageux d'utiliser un adénovirus recombinant défectif comprenant un ADN double-brin de l'invention inséré dans un gène VA.

Par ailleurs, la faible taille des ADN. double-brin de l'invention permet de manière avantageuse d'incorporer simultanément, dans un même vecteur, plusieurs de ces ADN, identiques (dirigés contre le même acide nucléique-cible) ou différents (dirigés contre des acides nucléiques-cible différents). Un mode de réalisation particulier de l'invention consiste donc dans un vecteur, notamment viral, comprenant au moins deux ADN double-brin tels que définis ci-dessus.

Les virus recombinants défectifs de l'invention peuvent être préparés par recombinaison homologue entre un virus défectif et un plasmide portant entre autre l'ADN double-brin tel que défini ci-avant (Levrero et al., Gene 101 (1991) 195; Graham, EMBO J. 3(12) (1984) 2917). La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus ou d'AAV recombinants défectifs, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12%). A titre d'exemple de lignée utilisable pour la préparation de rétrovirus recombinants défectifs, on peut mentionner la lignée CRIP (Danos et Mulligan, PNAS 85 (1988) 6460).

Ensuite, les virus qui se sont multipliés sont récupérés et purifiés selon les techniques classiques de biologie moléculaire.

La présente invention a également pour objet une composition pharmaceutique comprenant au moins un vecteur ou un ADN double-brin tels que définis ci-dessus.

Les compositions pharmaceutiques de l'invention peuvent être formulées en vue d'une administration par voie topique, orale, parentérale, intranasale, intraveineuse, intramusculaire, sous-cutanée, intraoculaire, etc.

Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Les doses d'ADN (ou de vecteur) utilisées pour l'administration peuvent être adaptées en fonction de différents paramètres, et notamment en fonction du mode d'administration utilisé, de la pathologie concernée, de l'acide nucléique à exprimer, ou encore de la durée du traitement recherchée. D'une manière générale, concernant les virus recombinants selon l'invention, ceux-ci sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

De telles compositions pharmaceutiques peuvent être utilisées chez l'homme, pour le traitement et/ou la prévention de maladies résultant d'une expression anormale de gènes (surexpression d'un gène cellulaire, expression d'un gène muté, etc), ou de maladies virales (HIV, herpes, hépatite, etc).

La présente invention a également pour objet un procédé de régulation de l'expression de gènes dans une cellule déterminée comprenant l'introduction dans ladite cellule d'un ADN double-brin tel que défini ci-avant. Cet ADN peut être administré tel quel ou, comme décrit ci-avant, après son incorporation dans un vecteur. Ce procédé de l'invention est tout particulièrement utilisable dans le cadre d'une thérapie cellulaire, pour modifier *ex vivo* l'expression de gènes sur des populations cellulaires déterminées, prélevées à partir d'un organisme, et avant leur réadministration. Il peut s'agir d'un gène dont les niveaux d'expression sont modifiés dans la pathologie concernée (ex. oncogène, gène viral), ou d'un gène dont le niveau d'expression n'est pas affecté, mais participe au développement de ladite pathologie.

La présente invention a également pour objet un procédé de régulation de l'expression de gènes *in vivo* comprenant l'administration d'une composition pharmaceutique telle que définie ci-avant

La présente invention a également pour objet une méthode de traitement de maladies résultant d'une expression anormale de gènes comprenant l'administration d'une composition pharmaceutique telle que définie ci-avant comprenant un ADN double-brin codant pour un ARN capable de modifier l'expression desdits gènes.

La présente invention sera plus complètement décrite à l'aide des exemples qui suivent, qui doivent être considérés comme illustratifs et non limitatifs.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC, pSP65 et les phages de la série M13 sont d'origine commerciale (Bethesda Research Laboratories, Promega).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée *in vitro* par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemple 4: Synthèse d'ADN double-brin codant pour des ARN triple-hélice dirigés contre le gène IGF-I (Insulin-like Growth Factor I).

Cet exemple décrit la synthèse de différents ADN double-brin codant pour des ARN triple-hélice dirigés contre le gène IGF-I, au niveau d'une région comprise entre les résidus 40 et 62 (SEQ ID n° 16).

Cette région du gène IGF-I identifiée ci-dessus comprend 23 bases puriques, et est localisée dans la partie 5' transcrite mais non traduite du gène.

Différents ADN double-brin codant pour des ARN triple-hélice selon l'invention dirigés contre cette cible ont été synthétisés. La synthèse a été réalisée au moyen d'un synthétiseur automatique de nucléotides, en utilisant la chimie des phosphoramidites selon la technique décrite par Giovannangeli et al. (J. Am. Client. Soc. 113 (1991) 7775). La séquence de ces ADN est donnée dans le tableau ci-apres.

| **CIBLE IGF-I** SEQ ID n° 16 | 5'-     AGAAGAGGGAGAGAGAGAGAAGG ...-3' 40                    62 | | |
|---|---|---|---|
| **ADN db** | **2ème région** | **Bras** | **1ère région** |
| | 5' | | 3' |
| SEQ ID n° 17 | TCTTCTCCC(TC)₅TTCC | TCCG | CCTT(CT)₅CCCTCTTCT |

## Revendications

1. ADN double-brin codant pour un ARN capable de former une triple-hélice avec un acide nucleique-cible simple-brin codant pour IGF-1.

2. ADN double-brin selon la revendication 1, caratérisé en ce que l'ARN capable de former une triple hélice est dirigé contre le gène IGF-I au niveau d'une région comprise entre les résidus 40 et 62 (SEQ ID N°16)

3. ADN double-brin selon la revendication 2, **caractérisé en ce qu'**il comprend la séquence SEQ ID N°17

4. Vecteur d'expression comprenant un ADN double-brin selon l'une des revendications précédentes.

5. Vecteur selon la revendication 4, **caractérisé en ce qu'**il s'agit d'un virus recombinant.

6. Vecteur selon la revendication 5, **caractérisé en ce que** le virus est choisi parmi les adénovirus, les rétrovirus, les virus adéno-associés, le virus de l'herpès, le cytomégalovirus et le virus de la vaccine.

7. Vecteur selon l'une des revendications 5 ou 6, **caractérisé en ce qu'**il s'agit d'un virus défectif.

8. Composition pharmaceutique comprenant au moins un ADN double-brin ou un vecteur selon l'une des revendications précédentes.

9. Composition pharmaceutique comprenant au moins un ADN double-brin selon l'une des revendications 1 à 3, sous forme complexée à du DEAE-dextran, à des protéines nucléaires ou à des lipides, sous forme brute ou encore incorporée à des liposomes ou à des nanoparticules.

## Claims

1. Double-stranded DNA encoding an RNA capable of forming a triple helix with a region between residues 40 and 62 (SEQ ID No. 16) of a single-stranded target nucleic acid encoding IGF-1.

2. Double-stranded DNA according to Claim 1, **characterized in that** it comprises the sequence SEQ ID No. 17.

3. Expression vector comprising a double-stranded DNA according to either of the preceding claims.

4. Vector according to Claim 3, **characterized in that** it is a recombinant virus.

5. Vector according to Claim 4, **characterized in that** the virus is selected from adenoviruses, retroviruses, adeno-associated viruses, the herpesvirus, the cytomegalovirus and the vaccinia virus.

6. Vector according to either of Claims 4 and 5, **characterized in that** it is a defective virus.

7. Pharmaceutical composition comprising at least one double-stranded DNA or one vector according to one of the preceding claims.

8. Pharmaceutical composition comprising at least one double-stranded DNA according to either of Claims 1 and 2, in a form complexed with DEAE-dextran, with nuclear proteins or with lipids, in crude form or else in a form incorporated into liposomes or into nanoparticles.

## Patentansprüche

1. Doppelsträngige DNA, die eine RNA codiert, die in der Lage ist, eine Tripelhelix in einer Region zwischen den Resten 40 und 62 (SEQ ID NR: 16) einer einzelsträngigen Zielnukleinsäure, die IGF-1 codiert, zu bilden.

2. Doppelsträngige DNA nach Anspruch 1, **dadurch gekennzeichnet, dass** sie die Sequenz SEQ ID NR: 17 umfasst.

3. Expressionsvektor, umfassend eine doppelsträngige DNA nach einem der vorstehenden Ansprüche.

4. Vektor nach Anspruch 3, **dadurch gekennzeichnet, dass** es sich um ein rekombinantes Virus handelt.

5. Vektor nach Anspruch 4, **dadurch gekennzeichnet, dass** das Virus aus Adenoviren, Retroviren, adenoassoziierten Viren, dem Herpesvirus, dem Cytomegalievirus und dem Vakzinevirus ausgewählt ist.

6. Vektor nach einem der Ansprüche 4 oder 5, **dadurch gekennzeichnet, dass** es sich um ein defektives Virus handelt.

7. Pharmazeutische Zusammensetzung, umfassend mindestens eine doppelsträngige DNA oder einen Vektor nach einem der vorstehenden Ansprüche.

8. Pharmazeutische Zusammensetzung, umfassend mindestens eine doppelsträngige DNA nach einem der Ansprüche 1 oder 2 in einer mit DEAE-Dextran, nukleären Proteinen oder Lipiden komplexierten Form, in einer rohen oder auch in einer in Liposomen oder Nanopartikel eingebauten Form.
